# EUROPEAN PATENT APPLICATION

(11) **EP 1 704 871 A1**
(43) Date of publication of application: **27.09.2006**
(21) Application number: 05703552.9
(22) Date of filing: 13.01.2005
(51) Int. Cl.: A61K 45/00, A61K 38/12, A61P 7/06, A23L 1/304

(54) **IRON SUPPLEMENT AND UTILIZATION OF THE SAME**

(30) Priority: 14.01.2004 JP 2004007278
(71) Applicant: Gekkeikan Sake Co., Ltd., Fushimi-ku Kyoto-shi Kyoto 6128660 (JP)
(72) Inventor: SUZUKI, S. Research Inst. Gekkeikan Sake Co., Ltd., Kyoto-shi, Kyoto 6128385 (JP); FUKUDA, K. Research Inst. Gekkeikan Sake Co., Ltd., Kyoto-shi, Kyoto 6128361 (JP); IRIE, M. Research Inst. Gekkeikan Sake Co., Ltd., Kyoto-shi, Kyoto 6128361 (JP); HATA, Yoji Research Inst. Gekkeikan Sake Co., Ltd., Kyoto-shi, Kyoto 6128385 (JP); KAWATO, A. Research Inst. Gekkeikan Sake Co., Ltd., Kyoto-shi, Kyoto 6128385 (JP); ABE, Yasuhisa Gekkeikan Sake Co., Ltd., Kyoto-shi, Kyoto 6128361 (JP)
(74) Representative: Müller-Boré & Partner Patentanwälte
(86) International application number: PCT/JP2005/000313
(87) International publication number: WO 2005/067970

(57) **Abstract**

The present inventors have found that a siderophore-iron (III) ion chelate complex is highly absorbed in the body; significantly increases the blood hemoglobin concentration, the serum iron concentration and the concentration of iron stored in the liver; and causes no adverse effects on the body. On the basis of these findings, the present inventors provide iron supplementing agents, agents for the prevention or treatment of iron defeciency anemia, food additives and food compositions, each of which contains a siderophore and iron (III) ions, preferably in the form of a chelate complex.

## Description

### TECHNICAL FIELD

The present invention relates to iron supplementing agents, agents for preventing or treating iron deficiency anemia, food additives, and food compositions, each comprising a siderophore.

### BACKGROUND ART

In recent years, Japanese people have become increasingly conscious of their diet and health, and are demanding safety and functionality in foods. The proportion of people who take nutritional supplements has also increased in order to make up for nutrients lacking in their daily diet. According to a national nutrition survey, the average consumption of iron has continued to remain below the necessary amount, regardless of sex and age. It is also known that most of the iron consumed is lost from the body because of its poor absorption. For these reasons, iron is one of the nutrients that must be taken frequently.

If iron deficiency continues for a long period, this may cause not only iron deficiency anemia but also various adverse effects on the human body, such as decreased physical and learning abilities, and reduced immunity. In general, an effective approach to improve iron deficiency anemia is to consume meat and fish that are rich in heme iron, which is readily absorbed by humans. However, in order to consume a large amount of meat and fish, a major dietary change is necessary, and excessive consumption of such foods results in a poor nutrition balance. Iron contained in plants such as vegetables is non-heme iron, which is absorbed only about half to about one-fifth as much as heme-iron. The absorption of non-heme iron also tends to be inhibited by dietary fiber and catechins contained in green tea, etc. It is, therefore, not realistic to consume a sufficient amount of iron from plants.

A variety of iron-containing preparations such as iron pyrophosphate, iron citrate and iron sulfate, have been used to supplement iron for the prevention or treatment of anemia. However, iron contained in such preparations typically has low availability in the body, and hence needs to be consumed in a large amount to achieve an effect of preventing or treating anemia. It is, however, difficult to consume a large amount of such iron salts in a short period because of the risk of causing gastrointestinal mucosal disorders, vomiting, etc. Accordingly, such iron salts need to be intaken over a long period to achieve an effect of preventing or treating anemia.

Lactoferrin contained in milk has also been mentioned as another example of a conventional iron supplement. Lactoferrin is a sugar protein known to be capable of binding to iron. Patent document 1 discloses a method of preparing an iron-lactoferrin complex and a blood increasing effect provided by such a complex. The iron-lactoferrin complex, however, has many problems to be solved before it can become available as an iron supplement, because it has poor solubility and requires a large amount of lactoferrin per necessary amount of iron.

Moreover, non-patent document 1 discloses the results of research on the absorption of iron-fortifying preparations which contain EDTA-FeNa as an active ingredient. However, there is a health-related concern for the long-term consumption of such iron-fortifying preparations containing EDTA-FeNa, because EDTA, which strongly chelates many trace metals in the body, has the possibility of inactivating metals necessary in the body by chelation.

In the brewing of sake, a kind of mold, *Aspergillus oryzae* is grown on steamed rice to prepare *"koji",* which is used as a sub-ingredient. It has long been known that, during *koji* preparation, *A. oryzae* produces abundant ferrichromes, mainly desferri-ferrichrysin, and such ferrichromes chelate iron ions originating in the water for making sake, and the chelate complex is responsible for coloring of sake. *A. oryzae* is thus known to produce abundant ferrichromes, in sake brewing. Ferrichromes deteriorate the quality of sake, and therefore no effective use thereof has been expected.
[Patent Document 1] Japanese Patent No. 2884045
[Non-Patent Document 1] " Trace Nutrients Research ", vol. 18, pp. 25-28, (2001)

### DISCLOSURE OF THE INVENTION

### PROBLEM TO BE SOLVED BY THE INVENTION

An object of the present invention is to provide agents for supplementing iron which allow iron to be highly absorbed in the body, and agents for preventing or treating iron deficiency anemia, as well as food additives and food compositions.

### MEANS FOR SOLVING THE PROBLEM

The present inventors conducted extensive research to overcome the aforementioned object, thereby obtaining the following findings:
(1) Anemia due to iron deficiency develops through the three stages: 1) lack of iron stored in the liver, 2) lack of iron in the blood serum, and 3) decreased ability of synthesizing hemoglobin, which causes symptoms of anemia. Iron deficiency anemia recovers through these stages in the reverse order.
   When a siderophore-iron (III) chelate complex is intaken by a rat suffering from iron deficiency anemia, the complex is efficiently absorbed by the rat to increase the iron concentration in the serum and the hemoglobin concentration in the blood, so as to further increase iron stored in the liver. Therefore, the siderophore-iron (III) chelate complex is useful in preventing, improving, or treating various symptoms caused by iron deficiency, particularly iron deficiency anemia.
(2) The siderophore-iron (III) chelate complex, when intaken by a rat, does not adversely affect the body weight gain, food intake, and liver and kidney functions, thus providing a safe iron supplementing agent.
(3) The siderophore-iron (III) chelate complex is highly soluble in water, and shows high water solubility even in acidic conditions in which some iron compounds may precipitate, and can therefore be readily used as an additive in a liquid food.
(4) The siderophore-iron (III) chelate complex is very stable against heat and pressure over a wide pH range, and does not change its properties. Therefore, when the complex is used as an ingredient of pharmaceutical or food compositions, such compositions can be subjected to heat and/or pressure sterilization.
(5) A liquid-food composition comprising 0.05 to 10 mg per ml of the siderophore-iron (III) chelate complex is capable of effectively preventing or improving iron deficiency anemia on a moderate intake schedule without any side effects.
(6) A solid-food composition comprising 0.1 to 5 mg per g of the siderophore-iron (III) chelate complex is capable of effectively preventing or improving iron deficiency anemia on a moderate intake schedule without any side effects.

The present invention was accomplished based on the aforementioned findings, and provides iron supplementing agents and the like as summarized below.

Item 1. An iron supplementing agent comprising a siderophore and iron (III) ions.

Item 2. An iron supplementing agent according to Item 1, wherein a siderophore and iron (III) ions are contained in the form of a chelate complex.

Item 3. An iron supplementing agent according to Item 1 or 2, wherein the siderophore incorporates hydroxamic acids.

Item 4. An iron supplementing agent according to Item 3, wherein the chelate complex is a ferrichrome.

Item 5. An iron supplementing agent according to Item 4, wherein the ferrichrome is ferrichrysin.

Item 6. An agent for preventing or treating iron deficiency anemia, comprising a siderophore and iron (III) ions.

Item 7. An agent for preventing or treating iron deficiency anemia according to Item 6, wherein a siderophore and iron (III) ions are contained in the form of a chelate complex.

Item 8. An agent for preventing or treating iron deficiency anemia according to Item 6 or 7, wherein the siderophore incorporates hydroxamic acids.

Item 9. An agent for preventing or treating iron deficiency anemia according to Item 8, wherein the chelate complex is a ferrichrome.

Item 10. An agent for preventing or treating iron deficiency anemia according to Item 9, wherein the ferrichrome is ferrichrysin.

Item 11. A food additive for iron supplementation comprising a siderophore and iron (III) ions.

Item 12. A food additive for preventing or improving iron deficiency anemia comprising a siderophore and iron (III) ions.

Item 13. A food composition comprising a siderophore and iron (III) ions.

Item 14. A food composition according to Item 13, wherein a siderophore and iron (III) ions are contained in the form of a chelate complex.

Item 15. A food composition according to Item 13 or 14, wherein the food composition is in solid form, and contains 0.1 to 5 mg/g of the siderophore and iron (III) ions in terms of a chelate complex.

Item 16. A food composition according to Item 13 or 14, wherein the food composition is in liquid form, and contains 0.05 to 10 mg/ml of the siderophore and iron (III) ions in terms of a chelate complex.

Item 17. A food composition according to Item 16, wherein the food is a beverage selected from the group consisting of liquors, teas, coffees, sports drinks, refreshment drinks, dairy drinks, and soups.

Item 18. A food composition according to any one of Items 13 to 17 for use in iron supplementation.

Item 19. A food composition according to any one of Items 13 to 17 for use in preventing or improving iron deficiency anemia.

Item 20. A food composition according to any one of Items (i) to (vi) below, each comprising a siderophore and iron (III) ions:
(i) a food composition which has the function of supplementing iron, and carries an indication specifying a use for supplementing iron;
(ii) a food composition which has the function of assisting iron, and carries an indication specifying a use for assisting iron;
(iii) a food composition which has the function of fortifying iron, and carries an indication specifying a use for fortifying iron;
(iv) a food composition which has the function of adding iron, and carries an indication specifying a use for adding iron;
(v) a food composition which has the function of maintaining bodily iron content at a normal level, and carries an indication specifying a use for maintaining bodily iron content at a normal level; and
(vi) a food composition which has the function of eliminating or alleviating iron deficiency in the body, and carries an indication specifying a use for eliminating or alleviating iron deficiency in the body.

Item 21. An iron supplementing method comprising administering a composition containing a siderophore and iron (III) ions to a human.

Item 22. An iron supplementing method according to Item 21, wherein 20 to 170 mg of a siderophore and iron (III) ions in terms of a chelate complex is administered per day.

Item 23. A method of preventing or treating iron deficiency anemia comprising administering a composition containing a siderophore and iron (III) ions to a human.

Item 24. A method of preventing or treating iron deficiency anemia according to Item 23, wherein 20 to 170 mg of a siderophore and iron (III) ions in terms of a chelate complex is administered per day.

Item 25. A method of adding a composition containing a siderophore and iron (III) ions to a food.

Item 26. Use of a composition containing a siderophore and iron (III) ions as an iron supplementing agent.

Item 27. Use of a composition containing a siderophore and iron (III) ions as an agent for preventing or treating iron deficiency anemia.

Item 28. Use of a composition containing a siderophore and iron (III) ions as a food additive for iron supplementation.

Item 29. Use of a composition containing a siderophore and iron (III) ions as a food additive for preventing or improving iron deficiency anemia.

Item 30. Use of a composition containing a siderophore and iron (III) ions as a food composition for iron supplementation.

Item 31. Use of a composition containing a siderophore and iron (III) ions as a food composition for preventing or improving iron deficiency anemia.

### EFFECTS OF THE INVENTION

Iron (II) ions have heretofore been considered as being more absorbable by the body than iron (III) ions. However, the present inventors have found that siderophore-iron (III) chelate complexes are effectively absorbed by the body to increase the iron concentration in the serum, hemoglobin concentration in the blood, and concentration of iron stored in the liver.

Therefore, a composition containing a siderophore and iron (III) ions can be suitably used as an iron supplementing agent or a medicinal preparation, for example, for the prevention or treatment of iron deficiency anemia. Moreover, such a composition is capable of supplementing iron to prevent or improve iron deficiency anemia. Furthermore, such a composition may be added to a food to impart an iron supplementation effect or an effect of preventing or improving iron deficiency anemia to the food.

Since siderophore-iron chelate complexes are inherently contained in foods using koji, such as sake and sake lees, their safety has historically been proven. The complexes have also been proved harmless to the body in that they do not reduce liver and kidney functions, body weight gain, food intake and the like, as described below. The complexes are, therefore, suitable not only for improving or treating various symptoms of iron deficiency, but also for continuous use in order to prevent iron deficiency. They are also suitable for use in functional foods or foods for specific health uses consumed by healthy individuals.

Further, siderophore-iron (III) chelate complexes which are naturally contained in foods using koji do not have a strong taste or odor, and are therefore easily used as ingredients of pharmaceutical products or foods, particularly as food additives.

These complexes are easily dissolved in water, and are therefore readily made into syrups and similar types of agents, and can also be readily used as additives for liquid foods. The complexes can also be added to acidic liquid foods such as dressings because of their good solubility in low-pH solutions. Unlike some iron compounds which may precipitate from solutions having pHs of not more than 4, one of the characteristics of the siderophores is that they show high solubility in such acidic solutions.

The siderophores also show resistance to treatment at high temperatures and/or pressures, and do not or hardly change their properties by sterilization at high temperatures and/or pressures. Therefore, the siderophores can be subjected to heat and/or pressure sterilization when used as an ingredient in pharmaceutical products or foods. In particular, because of their resistance to pressure treatment, siderophores can also be utilized as an additive to retortable foods, which require strict sterilization conditions. Moreover, because of their resistance to heat and pressure over a wide pH range, the addition of siderophores to medicines and foods does not restrict the manufacturing processes, thus allowing diverse manufacturing processes.

In general, a complex is more likely to be a low energy state, i.e., chemically stable state, than the state in which a ligand and a metal ion are present separately. Therefore, a siderophore does not act to promote peroxide-producing reactions which are observed for iron ions *(*Metal. Ions Biol. Syst., Vol 35, p 37). Siderophores hence have high potential for use in foods and medical products, since they do not promote peroxide-producing reactions harmful to the body.

Furthermore, siderophore-iron (III) chelate complexes hardly become insoluble even in the presence of substances such as phytic or tannic acid, which are the food components known to usually insolubilize iron. Accordingly, siderophores and iron (III) ions can be included in any kinds of foods to efficiently achieve the effect of supplementing iron or effect of preventing or treating iron deficiency anemia.

### BEST MODE FOR CARRYING OUT THE INVENTION

The present invention is described in further detail below.

### (I) Iron Supplementing Agent and Agent for Preventing or Treating Iron Deficiency Anemia

An iron supplementing agent and an agent for preventing or treating iron deficiency anemia according to the present invention contains a siderophore and iron (III) ions, particularly as active ingredients. Siderophore and iron (III) ions may be present on their own or in the form of a chelate complex.

### Siderophores

The term "siderophores" encompasses compounds capable of chelating iron (III) ions.

Siderophores may be derived from any living organism without particular limitation. Many microorganisms produce siderophores to efficiently intake iron, an essential nutrient, in low iron-concentration environments. Siderophores can be easily mass-produced using microorganisms, which can be easily propagated. In this respect, siderophores derived from microorganisms are preferable. Siderophores from microorganisms are also preferable because hosts for producing abundant siderophores can be easily prepared by recombination of genes involved in siderophore biosynthesis.

Any kind of siderophores from microorganisms are useful. Examples of siderophore-iron (III) ion chelate complexes include catechols such as enterobactin, vibriobactin, agrobactin and anguibactin; hydroxamates such as coprogen, ferrichromes, ferrioxamine and N,N',N''-triacetyl fusarinine C; and polycarboxylates such as rhizoferrine.

Hydroxamates (i.e., siderophores incorporating hydroxamic acids), in particular, are preferable because of their good iron-chelating capabilities. Amongst hydroxamates, cyclic ferrichromes are more preferable because of their good stability.

The term "ferrichromes" herein collectively refers to cyclic peptide compounds incorporating three hydroxamic acids, and encompasses compounds represented by general formula (1) shown below: 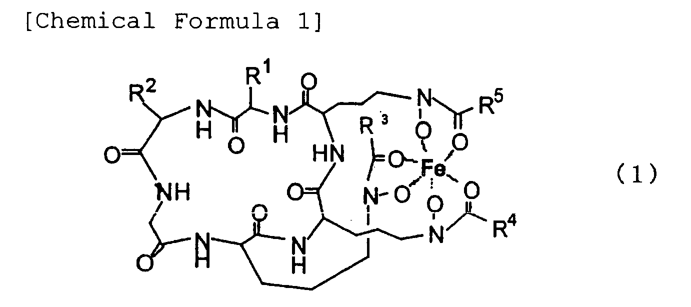 wherein R¹ represents a hydrogen atom or a hydroxymethyl group; R² represents a hydrogen atom, a methyl group, or a hydroxymethyl group; and R³, R⁴, and R⁵ are the same or different, each representing a methyl group, N⁵⁻(trans-5-hydroxy-3-methylpent-2-enoyl) group, N⁵-(cis-5-hydroxy-3-methylpent-2-enoyl) group, or N⁵-(trans-4-carboxy-3-methylpent-2-enoyl) group.

Preferable compounds among those which can be represented by general formula (1) are ferrichrome, diglycyl ferrichrome, ferrichrysin, ferrichrome C, ferricrocin, asperchrome D1, asperchrome B1, ferrirubin, ferrirhodin, ferrichrome A, and des(diserylglycyl)ferrirhodin (i.e., a compound obtained by removing Ser-Ser-Gly- from ferrirhodin). Of the above-mentioned compounds, ferrichrysin is the most preferable.

Table 1 below shows R¹ to R⁵, the functional groups in general formula (1), for each of these compounds.

**[Table 1]**

| | R¹ | R² | R³ | R⁴ | R⁵ |
|---|---|---|---|---|---|
| ferrichrome | H | H | CH₃ | CH₃ | CH₃ |
| (Gly)₄ ferrichrome* | H | H | CH₃ | CH₃ | CH₃ |
| ferrichrome C | H | CH₃ | CH₃ | CH₃ | CH₃ |
| ferricrocin | H | CH₂OH | CH₃ | CH₃ | CH₃ |
| ferrichrysin | CH₂OH | CH₂OH | CH₃ | CH₃ | CH₃ |
| asperchrome D1 | CH₂OH | CH₂OH | A | CH₃ | CH₃ |
| asperchrome B1 | CH₂OH | CH₂OH | CH₃ | A | A |
| ferrirubin | CH₂OH | CH₂OH | A | A | A |
| ferrirhodin | CH₂OH | CH₂OH | B | B | B |
| ferrichrome A | CH₂OH | CH₂OH | C | C | C |
| DDF** | - | - | B | B | B |

| | | | | | |
|---|---|---|---|---|---|
| * A compound resulting from ferrichrome incorporating four Gly molecules instead of three. ** des(diserylglycyl)ferrirhodin: a compound resulting from ferrirhodin lacking the three molecules Ser-Ser-Gly. A: N⁵-(trans-5-hydroxy-3-methylpent-2-enoyl) group B: N⁵-(cis-5-hydroxy-3-methylpent-2-enoyl) group C: N⁵-(trans-4-carboxy-3-methylpent-2-enoyl) group | | | | | |

The above-mentioned ferrichromes are produced by fungi such as *Aspergillus, Neurospora, Ustilago,* and the like species. *Aspergillus oryzae,* which is a filamentous fungus of the *Aspergillus* species, has been used in the production of sake, miso, soy sauce and the like. Humans have hence traditionally consumed ferrichromes. Ferrichromes are thus preferable in that their safety has been historically proven. Of the ferrichromes produced by koji fungi, ferrichrysin can be produced in relatively large amount by *Aspergillus oryzae,* thus being preferable because of its good productivity.

Siderophores for use in the present invention include natural siderophores and derivatives of natural siderophores capable of chelating iron (III). Examples of derivatives of natural siderophores include those resulting from acetylation, nitration, and substitution of some of the amino acids in natural siderophores.

Siderophores can be collected from living organisms. When living organisms are grown under iron-limiting conditions, desferri-compounds not containing iron (i.e., siderophores) are produced. Usually, iron (III)-chelate complexes are produced by adding iron to such desferri-compounds. Siderophores collected from living organisms may be employed in crude or purified form.

Siderophores may be purified, for example, by a known method such as any of a variety of chromatography processes, such as ion-exchange chromatography, hydrophobic chromatography, gel-filtration chromatography, affinity chromatography and the like. For example, when a siderophore is isolated from a filamentous fungus, the following methods may be used: the filamentous fungus may be incubated in a liquid medium to collect a siderophore produced in the supernatant of the liquid medium; or it may be incubated in solid medium to collect a siderophore from an extract obtained from the solid culture using water or buffer. In either case, the iron supplementing agent or agent for preventing or treating iron deficiency anemia according to the present invention may include, for example, impurities from living organisms.

Commercially available siderophores may also be used. Mixtures of siderophores may also be used.

### Ferrichrysin

Desferri-ferrichrysin, which is the ligand component of ferrichrysin, is a ferrichrome known to be abundantly produced from *Aspergillus oryzae* during the fabrication of koji (solid culturing). Most of the ferrichromes produced from *Aspergillus oryzae* are desferri-ferrichrysin. Desferri-ferrichrysin chelates iron (III) ions to form ferrichrysin.

Desferri-ferrichrysin is isolatable from *Aspergillus oryzae* by, for example, the following process. Examples of mediums useful for the growth of *Aspergillus oryzae* are potato dextrose medium (Nissui Inc.); a minimal medium (2% glucose (or starch), 0.3% NaNO₃, 0.2% KCl, 0.1% K₂HPO₄ and 0.05% MgSO₄; pH 6.0); and the like. Although the medium may be solid or liquid, a liquid medium is preferably used to facilitate the isolation of desferri-ferrichrysin. A solid medium such as rice koji is also preferable, because it can be directly used in a food composition without isolation and purification of desferri-ferrichrysin.

The incubation temperature may be in the range of temperatures which permit the growth of *Aspergillus oryzae,* for example, from about 25 to about 42°C. Although the incubation time varies depending on other conditions, it is usually from about 2 to about 7 days.

After the incubation has completed, the fungus is filtered, and subsequently ferrichrysin is collected from the culture supernatant. The culture supernatant is subsequently subjected to a known protein purification process, for example, any of a variety of chromatography processes, such as ion-exchange chromatography, hydrophobic chromatography, gel-filtration chromatography and affinity chromatography, so as to yield desferri-ferrichrysin in purified form.

### Iron (III) ions

Usually, a chelate complex is rapidly produced upon mixing a siderophore (i.e., desferri-compound) with an iron (III) ion. When the iron supplementing agent or agent for preventing or treating iron deficiency anemia is administered to a subject, iron (III) ions in the form of the chelate complex with the siderophore efficiently enhance the hemoglobin concentration in the blood, iron concentration in the serum, and iron concentration in the liver.

In the iron supplementing agent and agent for preventing or treating iron deficiency anemia, the molar ratio of the siderophore to iron (III) ions is usually preferable in the range of about 1:1 to about 5:1, and more preferable in the range of about 1:1 to about 2:1. Usually, it is most preferable that an eqimolar amount of the siderophore and iron (III) ions are present.

### Preparation Method

The iron supplementing agent and agent for preventing or treating iron deficiency anemia according to the present invention can be produced by mixing or combining a siderophore and iron (III) ions.

### Formulation

In formulating the iron supplementing agent or agent for preventing or treating iron deficiency anemia according to the present invention, a siderophore and iron (III) ions, preferably a siderophore-iron (III) ion chelate complex, are mixed with a variety of pharmacologically acceptable carriers (such as excipients, binding agents, disintegrators, lubricants, wetting agents and the like), so as to provide a suitable agent. The agent may further contain conventional additives.

The agent may take a variety of forms, and non-limiting examples of such forms include orally-administered agents such as tablets, pills, capsules, powders, granules and syrups; non-orally-administered agents such as injections, drops, agents for external use and suppositories; etc. Orally-administered agents are easier to use, because they impose a less burden on patients than non-orally-administered agents. In that respect, a siderophore and iron (III) ions, when combined for oral administration, are capable of effectively enhancing the concentrations of iron in the serum and blood, concentration of iron stored in the liver, and the like, and are therefore suitable for use as an agent in orally-administered form.

A wide range of known excipients can be used, and examples include various sugars such as lactose, sucrose and glucose; starches such as potato starch, wheat starch and corn starch; celluloses such as crystalline cellulose; and inorganic salts such as anhydrous calcium hydrogenphosphate and calcium carbonate; etc.

A wide range of known binding agents may be used, and examples include crystalline cellulose, pullulan, gum arabic, sodium alginate, polyvinylpyrrolidone, macrogol, etc.

A wide range of known disintegrators may be used, and examples include carboxymethylcellulose, calcium carboxymethylcellulose, hydroxypropylcellulose, hydroxypropyl starch, starches, sodium alginate, etc.

Examples of lubricants include magnesium stearate, talc, hydrogenated oils, etc.

A wide range of known wetting agents may be used, and examples include coconut oil, olive oil, sesame oil, peanut oil, soybean phospholipids, glycerol, sorbitol, etc.

The amount of the siderophore and iron (III) ions contained in the agent cannot be specified, because it varies depending on, e.g., the kind of siderophore, administration route, as well as the age, weight and symptoms of the subject or patient to which the agent is administered, etc. However, the amount may be that provides a siderophore and iron (III) ions dosage of from about 20 to about 170 mg, and preferably about 40 to about 80 mg, per day, in terms of a chelate complex, i.e. if all of the siderophore and iron (III) ions form chelate complexes. For administration once a day, the agent may contain aforementioned amount of the siderophore and iron (III) ions whereas for administration three times a day, the agent may contain one-third of this amount of the siderophore and iron (III) ions.

When the agent is in solid forms such as tablets, pills, capsules, powders, or granules, it may contain about 5 to about 30 wt% of a siderophore and iron (III) ions in terms of a chelate complex. When the agent is in liquid form such as a syrup, it may contain about 0.2 to about 1 wt% of a siderophore and iron (III) ions in terms of a chelate complex. When the agent is in injection or drop form, the agent may contain about 0.4 to about 2 wt% of a siderophore and iron (III) ions in terms of a chelate complex. For external use, the agent may contain about 1 to about 10 wt% of a siderophore and iron (III) ions in terms of a chelate complex. When the agent is a suppository, the agent may contain about 2 to about 20 wt% of a siderophore and iron (III) ions in terms of a chelate complex. When the content of the siderophore and iron (III) ions is within an aforementioned range, the effect of supplementing iron or effect of preventing or treating iron deficiency anemia can be sufficiently achieved without side effects.

Because siderophores do not or hardly change their properties by treatment at high temperatures (e.g., about 120°C) and/or pressures (e.g., about 200 kPa), they have the advantage of being able to be subjected to sterilization by heat and pressure for use as an ingredient in medicinal compositions. Moreover, because siderophores are traditionally contained in foods using koji, their safety has been historically proven. Accordingly, siderophores do not cause side effects when used as an ingredient of a pharmaceutical composition, or when used in an appropriate amount as an ingredient of a pharmaceutical composition.

### (II) Food Additives

The food additive according to the present invention is a food additive containing a siderophore and iron (III) ions. The siderophore and iron (III) ions may be contained in the form of a chelate complex. The additive can be advantageously used as an additive for imparting an effect of supplementing iron or effect of preventing or improving iron deficiency anemia to a food.

In addition to the siderophore and iron (III) ions, the food additive may also contain carriers such as sugars, starches, celluloses, magnesium stearate, vegetable oils and the like; and additives. When the food additive contains carriers and/or additives, the content of the siderophore and iron (III) ions in terms of a chelate complex may be about 0.4 to about 4 wt% when the food additive is in solid form; and the content may be about 0.04 to about 0.4 wt% when the food additive is in liquid form. The preferable ratio of the siderophore to iron

### (III) ions is the same as described for pharmaceutical agents.

Such food additives may take a variety of forms, and non-limiting examples include powders, granules, liquids and the like.

### (III) Food Compositions

The food composition according to the present invention is a composition containing a siderophore and iron (III) ions, preferably in the form of a chelate complex.

Such a food composition has the functions of supplementing iron (namely, assisting, fortifying and adding iron; maintaining bodily iron content at a normal level; and eliminating or reducing iron deficiency in the body), and preventing or improving iron deficiency anemia. Therefore, the food composition may have an indication which indicates a use for any of such purposes. The food composition can be suitably used as, for example, a functional food or food for a specific health use. The aforementioned complex is in this case contained as an active ingredient.

A siderophore-iron (III) complex is a composition traditionally contained in sake, and does not have a taste or odor which may impair food taste or flavor. Therefore, the complex can be applied to a variety of foods, and non-limiting examples of such foods include confectionaries such as candies, gums, cakes, pies, cookies, crackers, jellies, chocolates, puddings, ice creams, potato chips, sweetened and jellied adzuki-bean paste (*yokan*)*,* rice crackers (senbei), steamed filled dumplings (*manju*)*,* and Chinese steamed filled dumplings (*chuka-manju*)*;* beverages such as liquors, teas, coffees, sports drinks, refreshment drinks, soups, and dairy drinks; dairy products such as yogurt, butter and cheese; pastes such as hams, sausages, boiled fish pastes (*kamaboko*) and tubular fish meat (*chikuwa*); seasonings such as sauces, dressings, mayonnaise, soy sauce, soybean paste *(miso),* vinegar, sweet sake for seasoning *(mirin),* tomato based products (e.g., ketchup, tomato paste and tomato puree), curry roux, sake lees, and granular stocks; stock dishes such as seasoning powders for rice (*furikake*), pickles, shellfish boiled in sweetened soy sauce (*tsukudani*), and salted kelp; dishes; staple foods such as noodles, rice and rice porridge; and so forth.

Since siderophores are highly soluble in water, they are preferably used in liquid foods such as drinks and liquid seasonings such as sauces, dressings, soy sauce, vinegar, *mirin* and the like; and more preferably in drinks such as liquors, teas, coffees, sports drinks, refreshment drinks, dairy drinks and soups. The most preferable are liquors, teas, coffees, sports drinks, and refreshment drinks. Moreover, siderophores are highly soluble even in low-pH conditions wherein some iron compounds have lowered solubilities. Therefore, they have the advantage of being suitably used as additives for acidic liquid foods, such as mayonnaise and dressings. In addition, siderophores are resistant to treatment at high temperatures and pressures as described above. Therefore, foods containing the iron supplementing agent of the present invention can undergo sterilization by heat and pressure as well as special manufacturing processes such as retort processing. Furthermore, siderophores are suitable for use as food additives, because they are traditionally contained in foods using koji and have their safety historically proven.

The amount of the siderophore and iron (III) ions contained in the food composition of the present invention cannot be specified, because it varies depending on, e.g., the kind of siderophore as well as the age, weight and symptoms of the subject or patient to which the food composition is given, etc. However, the siderophore and iron (III) ions may be administered usually in an amount of about 20 to about 170 mg, and preferably about 40 to about 80 mg, per day in terms of a chelate complex.

The concentration of the siderophore and iron (III) ions contained in a food also depends on the kind of the food and the like. However, in the case of a solid food, the content of the siderophore and iron (III) ions in terms of a chelate complex is preferably from about 0.1 to about 5 mg/g, more preferably from about 0.2 to about 3 mg/g, and still more preferably from about 0.25 to about 1.5 mg/g. In the case of a liquid food, the content of the siderophore and iron (III) ions in terms of a chelate complex is preferably from about 0.05 to about 10 mg/ml, more preferably from about 0.1 to about 5 mg/ml, and still more preferably from about 0.2 to about 1 mg/ml. Usually, the amount of one serving for a solid food is from about 10 to about 50 g, and the amount of one serving for a liquid food is from about 50 to about 500 ml, although this depends on the forms of foods. Accordingly, the present food composition in an aforementioned range is capable of providing the amount of iron ions necessary per day. When the content of the siderophore and iron (III) ions is within an forementioned range, the effect of providing iron or the effect of preventing or improving iron deficiency anemia can be sufficiently achieved without the risk of hyperferremia.

When the content of the siderophore and iron (III) ions in the food composition, especially Japanese sake among liquid foods, is in an aforementioned range, the food composition works effectively for iron deficiency anemia without side effects, and without giving unpleasant feelings when being consumed.

The present food composition can additionally contain a variety of additives which are conventionally used for the preparation of food compositions. Examples of additives include stabilizers, pH adjusters, sugars, sweeteners, flavorings, a variety of vitamins, minerals, anti-oxidizing agents, excipients, solubilizers, binding agents, lubricants, suspending agents, wetting agents, coating materials, corrigents, colorants, preservatives and the like.

The present food composition is also suitable for use as a dietary supplement for iron supplementation or preventing or improving iron deficiency anemia. The dietary supplement may contain, in addition to the siderophore and iron (III) ions, known ingredient(s) as carrier(s) for the supplement. Examples of such carriers include sugars, starches, celluloses, magnesium stearate, vegetable oils and the like.

The amount of the siderophore and iron (III) ions contained in the supplement is preferably from about 30 to about 300 mg, and more preferably from about 60 to about 200 mg in terms of a chelate complex. When the chelate complex is within an aforementioned range, the effect of iron supplementation or effect of preventing or improving iron deficiency anemia can be sufficiently achieved, and side effects due to excessive intake can be avoided.

Such supplements may take a variety of forms, and non-limiting examples of forms include tablets, powders, granules and the like.

### (IV) Iron Supplementing Method and Method for Preventing, Improving or Treating Iron Deficiency Anemia

Because a siderophore-iron (III) chelate complexe allows iron to be highly absorbed in the body, the administration of a siderophore and iron (III) ions to a human makes it possible to supplement iron so as to effectively prevent, improve or treat decreased physical and/or learning abilities, reduced immunity and the like due to iron deficiency. The administration is also particularly effective in preventing, improving or treating iron deficiency anemia.

The iron supplementing agent or composition may be administered to both healthy individuals and individuals suffering from symptoms of iron deficiency. The iron supplementing agent and composition may also be suitably administered to growing children or adult women who frequently become iron deficient.

Although the amount of administration varies depending on, for example, the kind of siderophore, symptoms, age and weight of the subject to which the agent or composition is administered, etc., the iron supplementing agent or composition may be administered once or several times daily to provide the siderophore and iron (III) ions in an amount from about 20 to about 170 mg, and preferably from about 40 to about 80 mg, per day, in terms of a chelate complex.

Although the agent or composition may be administered both orally and non-orally, the oral administration is easier and preferable in that it can also effectively enhance the iron concentration in the serum.

### EXAMPLES

The present invention is further described in detail through the following Examples and test examples, which are not intended to limit the disclosure of the invention.

### Preparation of Ferrichrysin

An extract (containing desferri-ferrichrysin) obtained by extracting rice koji with water was mixed with an amount of ferric chloride equimolar to desferri-ferrichrysin to prepare a ferrichrysin solution. Macromolecules with molecular weights of 5000 or more such as proteins were subsequently removed from the solution through ultrafiltration membranes, after which the filtrate was concentrated by column chromatography. Ferrichrysin is known to show an absorption maximum at 430 nm *(*Agr. Biol. Chem., vol. 31, no. 12, p. 1482). Accordingly, through HPLC analysis of the solution prepared by the aforementioned process, most of the substances which showed an absorption maximum at 430 nm were confirmed to be ferrichrysin.

The concentration of all kinds of iron, i.e., ferrichrysin, water-soluble heme iron and ferric citrate, contained in foods given to rats was adjusted to 35 ppm utilizing not HPLC but atomic absorption. The HPLC analysis of ferrichrysin was a quantitative analysis which demonstrated that most of the substances absorbing at 430 nm were ferrichrysin.

### Example 1

Fifteen four-week-old SD male rats were freely fed an iron-deficient food for 35 days to develop iron deficiency anemia with the average blood hemoglobin value reduced to 6.5 g/dl. Five four-week-old SD male rats as the control group were fed iron-containing food 1. Iron-containing food 1 given to the control group contained ferric citrate as an iron source, which is typically used in rat foods, and had an iron content of 35 ppm, i.e., an optimal iron content for growing rats.

**[Table 2]**

| Contents | Iron-deficient food (%) | Iron-containing food 1 (%) |
|---|---|---|
| Corn starch | 33 | 33 |
| Casein | 22 | 22 |
| Cellulose powder | 5 | 5 |
| Sucrose | 30 | 30 |
| Corn oil | 5 | 5 |
| Salt mixture 1 | 4 | 0 |
| Salt mixture 2 | 0 | 4 |
| Vitamin mixture | 1 | 1 |
| Iron content | 1 ppm | 35 ppm |

Salt mixtures 1 and 2 shown in Table 2 above were prepared in accordance with the Harper salt mixture, and each had the composition shown in Table 3 below.

**[Table 3]**

| Contents | Salt mixture 1 (content (g) per 100 g) | Salt mixture 2 (content (g) per 100 g) |
|---|---|---|
| CaHPO₄·2H₂O | 0.43 | 0.43 |
| KH₂PO₄ | 34.31 | 34.31 |
| NaCl | 25.06 | 25.06 |
| Fe-citrate (Fe 17%) | 0 | 0.45 |
| MgSO₄ | 4.876 | 4.876 |
| ZnCl₂ | 0.02 | 0.02 |
| MnSO₄·4-5H₂O | 0.121 | 0.121 |
| CuSO₄·5H₂O | 0.156 | 0.156 |
| KI | 0.0005 | 0.0005 |
| CaCO₃ | 29.29 | 29.29 |
| (NH₄)₆MoO₂₄·4H₂O | 0.0025 | 0.0025 |

| | | |
|---|---|---|
| *Each mixture is adjusted to 100 g with cellulose powder.* | | |

The vitamin mixture shown in Table 2 was prepared in accordance with the AIN-76 Vitamin (choline addition), and had the composition shown in Table 4 below.

**[Table 4]**

| Contents | Content per 100 g |
|---|---|
| Vitamin A·acetate | 40,000 IU |
| Vitamin D₃ | 10,000 IU |
| Vitamin E·acetate | 500 mg |
| Vitamin K₃ | 0.5 mg |
| Vitamin B₁·hydrochrolide | 60 mg |
| Vitamin B₂ | 60 mg |
| Vitamin B₆·hydrochrolide | 70 mg |
| Vitamin B₁₂ | 0.1 mg |
| D-biotin | 2 mg |
| Folic acid | 20 mg |
| Calcium pantothenate | 160 mg |
| Nicotinic acid | 300 mq |
| Choline bitartrate | 20 g |

| | |
|---|---|
| *Each mixture is adjusted to 100 g with cellulose powder.* | |

The fifteen rats having iron deficiency anemia were subsequently divided into three groups, and were freely fed iron-containing foods 1, 2 and 3, respectively, for 3 weeks. As the iron source, iron-containing food 1 contains ferric citrate; iron-containing food 2 contains water-soluble heme iron; and iron-containing food 3 contains ferrichrysin. Table 5 below shows the compositions of iron-containing foods 1, 2 and 3. Iron-containing food 1 is the same as that shown in Table 2.

**[Table 5]**

| Contents | Iron-containing food 1 (%) | Iron-containing food 2 (%) | Iron-containing food 3 (%) |
|---|---|---|---|
| Corn starch | 33 | 33 | 33 |
| Casein | 22 | 22 | 22 |
| Cellulose powder | 5 | 5 | 5 |
| Sucrose | 30 | 30 | 30 |
| Corn oil | 5 | 5 | 5 |
| Salt mixture 2 | 4 | 0 | 0 |
| Salt mixture 3 | 0 | 4 | 0 |
| Salt mixture 4 | 0 | 0 | 4 |
| Vitamin mixture | 1 | 1 | 1 |
| Iron content | 35 ppm | 35 ppm | 35 ppm |

Salt mixtures 2, 3 and 4 shown in Table 5 have the compositionshown in Table 6 below. The vitamin mixture shown in Table 5 had the composition shown in Table 4 above.

**[Table 6]**

| Contents | Salt mixture 2 (content (g) per 100 g) | Salt mixture 3 (content (g) per 100 g) | Salt mixture 4 (content (g) per 100 g) |
|---|---|---|---|
| CaHPO₄·2H₂O | 0.43 | 0.43 | 0.43 |
| KH₂PO₄ | 34.31 | 34.31 | 34.31 |
| NaCl | 25.06 | 25.06 | 25.06 |
| Fe-citrate (Fe 17%) | 0.45 | 0 | 0 |
| Heme (Fe 1%) | 0 | 10.1 | 0 |
| Ferrichrysin (Fe 1%) | 0 | 0 | 11.5 |
| MgSO₄ | 4.876 | 4.876 | 4.876 |
| ZnCl₂ | 0.02 | 0.02 | 0.02 |
| MnSO₄·4-5H₂O | 0.121 | 0.121 | 0.121 |
| CuSO₄·5H₂O | 0.156 | 0.156 | 0.156 |
| KI | 0.0005 | 0.0005 | 0.0005 |
| CaCO₃ | 29.29 | 29.29 | 29.29 |
| (NH₄)₆MoO₂₄·4H₂O | 0.0025 | 0.0025 | 0.0025 |

| | | | |
|---|---|---|---|
| *Each mixture is adjusted to 100 g with cellulose powder* | | | |

### Measurement of Hemoglobin Concentration in the Blood

Blood was collected from the groups of rats which were fed iron-containing food 1 (ferric citrate), food 2 (heme iron) and food 3 (ferrichrysin) after the development of iron deficiency anemia both prior to and 3 weeks after giving these foods, so as to measure hemoglobin concentrations in the blood by a usual method. The rats in the control group were fed iron-containing food 1 (ferric citrate) during the study. The results are shown in Table 7 below. Table 7 shows the mean ± standard deviation value of hemoglobin concentrations of the five rats in each group.

**[Table 7]**

| | Before feeding iron-containing food (g/dl) | After feeding iron-containing food (g/dl) |
|---|---|---|
| Ferric citrate group | 5.7 ± 0.7 | 13.3 ± 2.3 |
| Water-soluble heme iron group | 6.1 ± 1.0 | 8.0 ± 0.9 |
| Ferrichrysin group | 6.4 ± 0.9 | 14.3 ± 0.3 |
| Control group | 14.8 ± 0.6 | 14.2 ± 0.1 |

As is evident from Table 7, the hemoglobin values of the groups which consumed ferric citrate and ferrichrysin, respectively, increased to the same level as that of the control group. In contrast, the group which consumed water-soluble heme iron showed little recovery of the blood hemoglobin concentration. After the feeding of the iron-containing foods, there was a significant difference between the heme-iron group and the ferrichrysin group at a significance level of 5%. As a result, the group which consumed ferrichrysin showed a therapeutic effect against the decreased ability of synthesizing hemoglobin, i.e., the third step of iron deficiency anemia, which was equal to that of the ferric citrate group, and was also significantly higher than that of the heme-iron group.

### Measurement of Serum Iron Concentration

Serum was prepared from the blood wholly exsanguinated from the rats of each group after the rats had been given their iron-containing food, so as to measure iron concentrations in the serums by a usual method. The results are shown in Table 8 below. Table 8 shows the mean ± standard deviation value of serum iron concentrations of the five rats in each group.

**[Table 8]**

| | After feeding iron-containing food (µg/dl) |
|---|---|
| Ferric citrate group | 131.5 ± 25.7 |
| Water-soluble heme iron group | 50.4 ± 6.6 |
| Ferrichrysin group | 116.7 ± 24.1 |
| Control group | 106.2 ± 4.7 |

As is evident from Table 8, the serum iron concentrations of the groups which consumed the foods containing ferric citrate and ferrichrysin, respectively, as the iron source, increased to the same level as that of the control group. In contrast, the iron concentration in the serum of the group which consumed water-soluble heme iron remarkably decreased, hence resulting in a significant difference from that of the control group at a significance level of 1%. Consequently, the group which consumed ferrichrysin showed a therapeutic effect against the decreased transport of iron, i.e., the second step of iron deficiency anemia, which was equal to that of the ferric citrate group, and was also significantly higher than that of the heme-iron group.

### Measurement of Iron Concentration in the Liver

After the induction of iron deficiency anemia, rats were fed iron-containing food 1 (ferric citrate), food 2 (heme iron) and food 3 (ferrichrysin),respectively, for three weeks. Rats of the control group were fed food 1 (ferric citrate) from the beginning, i.e. through to the end. At the end of the experiment, livers were removed after perfusion of saline into the portal vein to exclude the blood; they were weighed and freeze-dried. The dried liver was subsequently incinerated and subjected to atomic absorption, so as to measure the iron concentration in the liver. Further, the water content of the liver was calculated, and the concentration of iron stored in the liver of the body was determined based on the iron concentration in the dried liver and the water content of the liver. The results are shown in Table 9. Table 9 shows the mean ± standard deviation value of iron concentrations in the livers of the five rats in each group.

**[Table 9]**

| | Iron concentration in the liver (ppm) |
|---|---|
| Ferric citrate group | 21.6 ± 3.1 |
| Water-soluble heme iron group | 6.2 ± 1.3 |
| Ferrichrysin group | 39.3 ± 7.6 |
| Control group | 63.3 ± 10.1 |

As is evident from Table 9, the groups which were fed the iron-containing foods had developed severe iron deficiency anemia, and accordingly, the amounts of iron stored in the livers of all these groups were decreased as compared to the control group. However, significant differences were observed among these groups at a significant level of 1%, revealing which of the iron sources is more effective in increasing the amount of stored iron. Consequently, the group which consumed ferrichrysin showed a therapeutic effect against decreasing concentration of stored iron, i.e., the third step of iron deficiency anemia, which was significantly superior to the groups of ferric citrate and water-soluble heme iron.

### Effects on Liver and Kidney Functions

Measurements were made by a usual method of the concentrations of protein, ALT (alanine aminotransferase), AST (aspartate aminotransferase) and creatinine in the serums of the groups of rats which were fed iron-containing food 1 (ferric citrate), food 2 (heme iron) and food 3 (ferrichrysin) after the development of iron deficiency anemia, as well as the rats in the control group which were fed food 1 (ferric citrate) from the beginning. The results are shown in Table 10 below. Table 10 shows the values of mean ± standard deviation of the five rats in each group. There are significant differences between the values indicated by a and b.

**[Table 10]**

| | Serum protein (mg/ml) | ALT (Karmen) | AST (Karmen) | Creatinine (mg/dl) |
|---|---|---|---|---|
| Ferric citrate group | 69.5 ± 3.9 | 22.0 ± 3.5 | 96.0 ± 14.0^{a} | 0.57 ± 0.00 |
| Water-soluble heme iron group | 67.0 ± 1.8 | 23.7 ± 3.2 | 90.3 ± 8.5 | 0.75 ± 0.11 |
| Ferrichrysin group | 67.6 ± 4.1 | 17.7 ± 2.3^{a} | 72.3 ± 3.2^{b} | 0.63 ± 0.06 |
| Control group | 72.8 ± 5.8 | 29.3 ± 4.2^{b} | 69.0 ± 5.2^{b} | 0.71 ± 0.09 |

As is evident from Table 10, the serum analysis values for all the groups were within normal ranges, and there were no results indicating abnormality in the liver or kidney function. This suggests that the organ functions are not affected by the difference of iron sources. The group which consumed ferrichrysin as the iron source showed good results for all the examined items, revealing that the intake of ferrichrysin causes no harmful side effects on the body.

### Effects on Body Weight Gain and Food Intake

Table 11 shows the individual body weight gain and food intake of the rats in the groups which were fed iron-containing food 1 (ferric citrate), food 2 (heme iron) and food 3 (ferrichrysin) after the development of iron deficiency anemia, as well as the rats in the control group which were fed food 1 (ferric citrate) from the beginning. The weight gain in each group is the mean ± standard deviation value of the five rats in the group.

**[Table 11]**

| | Body weight gain (g/3 weeks) | Average food intake (g/3 weeks) |
|---|---|---|
| Ferric citrate group | 79.8 ± 9.2 | 384.8 |
| Water-soluble heme iron group | 55.3 ± 14.9 | 328.3 |
| Ferrichrysin group | 70.1 ± 8.3 | 366.3 |
| Control group | 65.3 ± 19.2 | 336.2 |

As is evident from Table 11, the weight gain of the group which consumed water-soluble heme iron was reduced, although there was no significant difference between the heme-iron group and the control group. Suppression of the weight gain and reduction in the food intake due to anemia seems to have occurred in the group which consumed water-soluble heme iron. In contrast, the weight gains and food intakes of the groups which consumed ferric citrate and ferrichrysin, respectively, increased as compared to those of the control group. The results suggest that the intake of ferrichrysin causes no harmful side effects, such as suppression of weight gain and eating disorders, on the growth of the body.

### Example 2

### Solubility of Ferrichrysin in Water

The solubility of ferrichrysin was compared with the solubilities of other iron compounds. Water-soluble heme iron, ferric citrate and ferrichrysin which were used in Example 1 were used as iron compounds. 0.1 g of each of these iron compounds was dissolved in 1 ml of each buffer having a pH of 2 or 7, and then the solutions were incubated at 37°C for 90 min. A glycine/hydrochloric acid buffer was used as the pH 2 buffer, and a phosphate buffer was used as the pH 7 buffer. The concentration of buffer agent(s) in each buffer was adjusted to 0.1 mol/l. After being incubated, each buffer was centrifuged to visually confirm the presence or absence of precipitate. Further, the iron concentration in the supernatant of each buffer was measured by atomic absorption. The results are shown in Table 12 below.

**[Table 12]**

| | Iron concentration | | Precipitate |
|---|---|---|---|
| Water-soluble heme iron | pH 2.0 | N.D. | Yes |
| | pH 7.0 | 468 | No |
| Ferric citrate | pH 2.0 | 600 | Yes |
| | pH 7.0 | 400 | Yes |
| Ferrichrysin | pH 2.0 | 3800 | No |
| | pH 7.0 | 3750 | No |

As is evident from Table 12, ferrichrysin showed very high water solubility. Some iron compounds are known to precipitate in acidic conditions. The results above show that ferric chloride and heme iron also precipitate in acidic conditions. In contrast, ferrichrysin showed high water-solubility even in acidic conditions. Although the iron in ferrichrysin is trivalent, it acquires such high water-solubility upon forming a complex. Consequently, the bioavailability of the iron improved.

### Example 3

### Thermal and pH Stabilities of Ferrichrysin

Ferrichrysin is known to show an absorption maximum at 430 nm. Ferrichrysin was tested for pH and thermal stabilities utilizing this property.

Ferrichrysin prepared from a rice koji extract in the manner as described above was dissolved in ultrapure water to a concentration of 2.5 mg/ml. The resulting solution was subsequently diluted to give a ferrichrysin concentration of 0.25 mg/ml using each of the following buffers. A glycinehydrochloric acid buffer was used for a pH 2 or pH 3 buffer; a acetate buffer was used for a pH 4 or pH 5 buffer; a phosphate buffer was used for a pH 6 or pH 7 buffer; a tris-hydrochloric acid buffer was used for a pH 8 buffer; and a glycine-sodium hydroxide buffer was used for a pH 9 or pH 10 buffer. The concentration of buffer agent(s) in each buffer was adjusted to 0.1 mol/l.

Each ferrichrysin solution was placed in a screw-cap test tube, and then the test tube was sealed. The solution was subsequently sterilized by a process of steaming under pressure for 20 min at a temperature of 120 °C and a pressure of 200 kPa. The absorbance of each solution at 430 nm was measured prior to and after the sterilization. The results are shown in Table 13 below.

**[Table 13]**

| pH | Absorbance at 430 nm | |
|---|---|---|
| | Before sterilization | After sterilization |
| 2.0 | 0.709 | 0.366 |
| 3.0 | 0.742 | 0.721 |
| 4.0 | 0.738 | 0.743 |
| 5.0 | 0.732 | 0.748 |
| 6.0 | 0.743 | 0.746 |
| 7.0 | 0.764 | 0.774 |
| 8.0 | 0.758 | 0.747 |
| 9.0 | 0.765 | 0.767 |
| 10.0 | 0.769 | 0.772 |

As is evident from Table 13, the ferrichrysin concentration in each ferrichrysin solution did not decrease over a wide pH range of from 3.0 to 10.0 after the sterilization in which the solution was subjected to high heat and pressure. The results establish that ferrichrysin has high thermal stability over a wide pH range of from 3.0 to 10.0.

### Example 4

### Reactivity of Ferrichrysin with Food Components

Iron compounds have the property of being rendered insoluble by binding to various components in foods, which is regarded as a cause of poor iron absorption. Ferrichrysin was tested for its reactivity with tannic acid and phytic acid, the typical iron uptake inhibitor, so as to compare its reactivity with that of other iron compounds with these iron uptake inhibitor.

Ferrichrysin prepared in the aforementioned manner, ferric citrate and sodium ferrous citrate were each independently dissolved in ultrapure water so that the iron concentration was 10 mg/ml. A 0.6% tannic acid solution and a 0.6% phytic acid solution were also prepared. The pH of each of these solutions was adjusted with hydrochloric acid or sodium hydroxide to 2.0, 4.0, 6.0 or 8.0.

The iron-compound solutions were mixed with each of the tannic acid and phytic acid solutions at a volume ratio of 1:9, and then the mixtures were incubated at 37 °C for 3 hr, so as to react the iron compounds with each of these iron uptake inhibitor. The resulting solutions were centrifuged at 15000 rpm for 5 min, and then the supernatant of each solution was ultrafiltrated using membranes having a molecular weight cut-off of 10000. The iron concentration in each filtrate was measured by atomic absorption. Table 14 below shows the proportion in % of the iron concentration after the reaction to the initial iron concentration (1 mg/ml) in each solution.

**[Table 14]**

| Phytic Acid | | | | |
|---|---|---|---|---|
| | pH 2.0 | pH 4.0 | pH 6.0 | pH 8.0 |
| Ferric citrate | 10.8 | 22.6 | 31.2 | 16.2 |
| Sodium ferrous citrate | 15.5 | 55.3 | 23.7 | 33.1 |
| Ferrichrysin | 85.7 | 100.6 | 101.8 | 100.3 |

| Tannic Acid | | | | |
|---|---|---|---|---|
| | pH 2.0 | pH 4.0 | pH 6.0 | pH 8.0 |
| Ferric citrate | 35.7 | 10.9 | 10.9 | 11.1 |
| Sodium ferrous citrate | 50.3 | 52.8 | 39.7 | 36.0 |
| Ferrichrysin | 80.5 | 71.8 | 76.1 | 81.9 |

As is evident from Table 14, ferrichrysin showed low reactivity with the two components which involve insolubilization of iron, and therefore retained high solubility of iron. This phenomenon was substantially consistent over the pH range of from 2.0 to 8.0, and the values of ferrichrysin were significantly higher than those of the other iron compounds. This can be attributed to the fact that ferrichrysin, which is an iron-containing complex, has a very high chemical stability, and therefore does not easily react with other food components. The results indicate that iron is efficiently absorbed even when ferrichrysin is consumed with a food having an iron-insolubilizing effect.

### Example 5

### Amounts of Ferrichrysin Added to Liquors (i.e., Liquid Foods)

Ferrichrysin was added to 100 ml of sake in amounts of 1, 5, 10, 20, 50, 100, 200, 500, and 1000 mg, so as to prepare iron-fortified sakes. The concentrations of ferrichrysin in the resulting iron-fortified sakes were 0.01, 0.05, 0.1, 0.2, 0.5, 1, 2, 5, and 10 mg/ml, respectively.

Sensory tests on each iron-fortified sake were conducted by 25 panelists. The results are shown in Table 15 below. In Table 15, +++ indicates that 20 or more panelists found the taste pleasant; ++ indicates that 7 to 19 panelists found the taste pleasant; + indicates that 1 to 6 panelists found the taste pleasant; and - indicates that no panelists found the taste pleasant.

**[Table 15]**

| Ferrichrysin concentration | 0.01 (mg/ ml) | 0.05 (mg/ ml) | 0.1 (mg/ ml) | 0.2 (mg/ ml) | 0.5 (mg/ ml) | 1 (mg/ ml) | 2 (mg/ ml) | 5 (mg/ ml) | 10 (mg/ ml) |
|---|---|---|---|---|---|---|---|---|---|
| | +++ | +++ | +++ | +++ | ++ | + | - | - | - |

As is evident from Table 15, good sensory evaluations were obtained when ferrichrysin was added to the sake in amounts of 1 mg/ml (0.1 wt%) or less, preferably 0.5 mg/ml (0.05 wt%) or less, and more preferably 0.2 mg/ml (0.02 wt%) or less.

### Example 6

### Amounts of Ferrichrysin Added to Cookies (i.e., Solid Foods)

Ferrichrysin was added to 100 g portions of cookies in amounts of 5, 10, 20, 50, 150, 300, 500, 1000, and 2000 mg, so as to prepare iron-fortified cookies. The concentrations of ferrichrysin in the resulting iron-fortified cookie portions were 0.05, 0.1, 0.2, 0.5, 1.5, 3.0, 5.0, 10, and 20 mg/g, respectively.

Sensory tests on each portion of the iron-fortified cookies were conducted by 25 panelists. The results are shown in Table 16 below. In Table 16, +++ indicates that 20 or more panelists found the taste pleasant; ++ indicates that 7 to 19 panelists found the taste pleasant; + indicates that 1 to 6 panelists found the taste pleasant; and - indicates that no panelists found the taste pleasant.

**[Table 16]**

| Ferrichrysin concentration | 0.05 (mg/ g) | 0.1 (mg/ g) | 0.2 (mg/ g) | 0.5 (mg/ g) | 1.5 (mg/ g) | 3.0 (mg/ g) | 5.0 (mg/ g) | 10 (mg/ g) | 20 (mg/ g) |
|---|---|---|---|---|---|---|---|---|---|
| | +++ | +++ | +++ | +++ | ++ | + | - | - | - |

As is evident from Table 16, good sensory evaluations were obtained when ferrichrysin was added to the cookies in amounts of 3 mg/g (0.3 wt%) or less, preferably 1.5 mg/g (0.15 wt%) or less, and more preferably 0.5 mg/g (0.05 wt%) or less.

### Formulation Examples

Formulation examples of food compositions according to the present invention are illustrated below.

### <Formulation Example 1: Cookies>

100 g of flour (soft flour), 2.5 g of baking powder, 1.5 g of salt and 200 mg of ferrichrysin were mixed, and then 40 g of butter and 50 g of milk were added to the mixture. The dough was baked in oven at 180 °C for 10 min, so as to obtain iron-fortified cookies. The cookies contained 1.5 mg/g of ferrichrysin.

### <Formulation Example 2: Jelly>

Swollen gelatin was prepared from 5 g of gelatin, 20 g of sucrose and 50 g of water. 160 mg of ferrichrysin was added to 140 g of plain yogurt. The swollen gelatin was added to the plain yogurt and was then cooled until set, so as to obtain an iron-fortified jelly. The jelly contained 0.75 mg/g of ferrichrysin.

### <Formulation Example 3: Candies>

500 g of highly refined sugar and 440 g of starch syrup were dissolved in a small amount of water. 4 g of ferrichrysin was added to the mixture, and then the mixture was boiled down at 130 °C under reduced pressure. 3.5g of citric acid, 1.5g of tartaric acid, and 1 g of defatted egg yolk-protein decomposition product were then added to the mixture. The resulting mixture was cooled to obtain iron-fortified candies. The candies contained 5 mg/g of ferrichrysin.

### <Formulation Example 4: Ice Cream>

1200 g of milk, 310 g of whipped cream, 300 g of highly refined sugar, 60 g of skim milk powder, 1 g of defatted egg yolk protein decomposition product, 6 g of thickener/stabilizer, and 800 mg of ferrichrysin were mixed with and dissolved in water to a total volume of 2000 ml. After the mixture was heated to 80°C, it was pre-emulsified and then homogenized by a homomixer. After being cooled and stood, the mixture was blended with 2 g of vanilla essence and was frozen. After that, it was rapidly frozen to -40°C to obtain an iron-fortified ice cream. The ice cream contained 0.4 mg/g of ferrichrysin.

### <Formulation Example 5: Yokan>

7.5 g of agar strips was dissolved in water and then mixed with 660 g of sweetened adzuki-bean paste, 300 g of granulated sugar, 550 ml of water and 2 g of ferrichrysin. The mixture was boiled down and cooled to obtain iron-fortified *yokan.* The *yokan* contained 2 mg/g of ferrichrysin.

### <Formulation Example 6: Yogurt>

20% skim milk was sterilized for 3 s, and then strains of *Streptococcus thermophilus* and *Lactobacillus casei* were incubated in the skim milk to obtain 400 g of yogurt base. 70g of sugar, 3 g of pectin and 800 mg of ferrichrysin were dissolved in water, and the total volume of the mixture was adjusted to 600 g by addition of water. The resulting mixture was sterilized at 120 °C for 3 s to obtain a syrup. After the yogurt base and syrup were mixed, 1 g of flavoring was added to the mixture. The mixture was then homogenized to obtain iron-fortified yogurt. The yogurt contained 0.8 mg/g of ferrichrysin.

### <Formulation Example 7: Milk>

100 ml of milk was mixed with 40 mg of ferrichrysin, and then the mixture was stirred well to obtain iron-fortified milk. The milk contained 0.4 mg/ml of ferrichrysin.

### <Formulation Example 8: Chocolate>

25 g of cacao mass, 15 g of cacao butter, 15g of whole milk powder, 30 g of powdered sugar, 15 g of powdered milk, and 250 mg of ferrichrysin were mixed together, and then the mixture was cooled after warming at 45 °C to obtain iron-fortified chocolate. The chocolate contained 2.5 mg/g of ferrichrysin.

### <Formulation Example 9: Mayonnaise>

20 g of egg yolk was mixed with 2.5 g of salt, 1.5 g of sucrose, 1.5 g of mustard, 0.1 g of pepper, 5 g of lemon juice, and 3.2 g of ferrichrysin. 10 g of vinegar and 160 g of salad oil were added to the mixture, and then the mixture was stirred well to obtain iron-fortified mayonnaise. The mayonnaise contained 16 mg/g of ferrichrysin.

### <Formulation Example 10: Ketchup>

1 kg of tomatoes were peeled, put into a mixer, and were then heated and boiled down. 10 g of grated onion and 3 g of garlic were added to the tomato paste, followed by addition of 10 g of sugar, 2 g of salt and 4.8 g of ferrichrysin. After reducing the heat to low, 1 g of spices (stick cinnamon, clove, pepper and capsicum) and 30 g of vinegar were added to the mixture. The mixture was heated once and cooled to obtain iron-fortified ketchup. The ketchup contained 16 mg/g of ferrichrysin.

### <Formulation Example 11: Curry Roux>

125 g of flour (soft flour), 100 g of butter, 20 g of curry powder and 1.0 g of ferrichrysin were mixed with addition of a small amount of water, and then the mixture was hardened to obtain iron-fortified curry roux. The curry roux contained 4 mg/g of ferrichrysin.

### <Formulation Example 12: Kamaboko>

100g of fish paste *(surimi),* 10 g of powdered mashed potatoes, 5 g of flour, 50 g of egg white, 2 g of salt, 0.2 g of sugar, 10 g of *mirin* and 1.3 g of ferrichrysin were mixed and made into a paste. The paste was then steamed in a steamer to obtain iron-fortified *kamaboko.* The *kamaboko* contained 7 mg/g of ferrichrysin.

### <Formulation Example 13: Tsukudani>

25 g of rehydrated kelp, 150 ml of bonito stock (katsuo-dashi), 15 g of sugar, 15 g of soy sauce, 10 g of vinegar and 400 mg of ferrichrysin were mixed. After the mixture was boiled once, it was brought to a simmer over medium heat to obtain iron-fortified *tsukudani.* The *tsukudani* contained 5 mg/g of ferrichrysin.

### <Formulation Example 14: Furikake>

15 g of toasted sesame, 20 g of seasoned sesame, 10 g of seasoned dried-bonito shavings, 10 g of seasoned dried-bonito containing salt granules, 10 g of seasoned dried-seaweed granules, and 1.6 g of ferrichrysin were mixed well to obtain iron-fortified *furikake.* The *furikake* contained 25 mg/g of ferrichrysin.

### <Formulation Example 15: Rice>

150 g of white rice mixed with 220 g of water and 160 g of ferrichrysin were cooked in a rice cooker to obtain iron-fortified rice. The rice contained 0.5 mg/g of ferrichrysin.

### <Formulation Example 16: Udon Noodles>

400 g of water and 50 g of salt were mixed until the salt was dissolved. 1000 g of all-purpose flour and 1.6 g of ferrichrysin were added to the mixture and kneaded well. When the dough had stiffened, it was well stretched and then cut into strips of 5-mm width. The dough was boiled for 10 min and then cooled to obtain iron-fortified *udon* noodles. The noodles contained 3 mg/g of ferrichrysin.

### <Formulation Example 17: Sake>

20 mg of ferrichrysin was added to 100 ml of sake, and then the mixture was stirred well to obtain iron-fortified sake. The sake contained 0.2 mg/ml of ferrichrysin.

### <Formulation Example 18: Shochu (Japanese distilled alcohol drink)>

40 mg of ferrichrysin was added to 100 ml of shochu, and then the mixture was stirred well to obtain iron-fortified shochu. The shochu contained 0.4 mg/ml of ferrichrysin.

### <Formulation Example 19: Wine>

20 mg of ferrichrysin was added to 100 ml of wine, and then the mixture was stirred well to obtain iron-fortified wine. The wine contained 0.2 mg/ml of ferrichrysin.

### <Formulation Example 20: Beer>

12 mg of ferrichrysin was added to 100 ml of beer, and then the mixture was stirred well to obtain iron-fortified beer. The beer contained 0.12 mg/ml of ferrichrysin.

### <Formulation Example 21: Whisky>

100 mg of ferrichrysin was added to 100 ml of whisky, and then the mixture was stirred well to obtain iron-fortified whisky. The whisky contained 1 mg/ml of ferrichrysin.

### <Formulation Example 22: Brandy>

100 mg of ferrichrysin was added to 100 ml of brandy, and then the mixture was stirred well to obtain iron-fortified brandy. The brandy contained 1 mg/ml of ferrichrysin.

### <Formulation Example 23: Spirits>

100 mg of ferrichrysin was added to 100 ml of spirits, and then the mixture was stirred well to obtain iron-fortified spirits. The spirits contained 1 mg/ml of ferrichrysin.

### <Formulation Example 24: Liqueur>

100 mg of ferrichrysin was added to 100 ml of a liqueur, and then the mixture was stirred well to obtain an iron-fortified liqueur. The liqueur contained 1 mg/ml of ferrichrysin.

### <Formulation Example 25: Mirin>

400 mg of ferrichrysin was added to 100 ml of *mirin,* and then the mixture was stirred well to obtain iron-fortified *mirin.* The mirin contained 4 mg/ml of ferrichrysin.

### <Formulation Example 26: Green Tea>

40 mg of ferrichrysin was added to 100 ml of green tea, and then the mixture was stirred well to obtain iron-fortified green tea. The green tea contained 0.4 mg/ml of ferrichrysin.

### <Formulation Example 27: Coffee>

40 mg of ferrichrysin was added to 100 ml of coffee, and then the mixture was stirred well to obtain iron-fortified coffee. The coffee contained 0.4 mg/ml of ferrichrysin.

### <Formulation Example 28: Sports Drink>

15 mg of ferrichrysin was added to 100 ml of water, 5 g of fructose, 2 g of sucrose, 0.3 g of citric acid, 20 mg of sodium, 2 mg of calcium, 20 mg of potassium, 20 mg of arginine, 10 mg of isoleucine, 10 mg of valine, 10 mg of leucine, 100 mg of vitamin C, and 1 mg of β-carotene, and the mixture was stirred well to obtain an iron-fortified sports drink. The sports drink contained 0.15 mg/ml of ferrichrysin.

### <Formulation Example 29: Refreshment Drink>

30 ml of Valencia orange juice, 3 ml of lemon juice, 1.5 g of fructose, 0.5 g of citric acid, and 0.1 g of vitamin C were added to 15 mg of ferrichrysin. The total volume of the mixture was adjusted to 100 ml by addition of water, and then the mixture was stirred well. Carbon dioxide was subsequently sealed in the mixture to obtain an iron-fortified refreshment drink. The refreshment drink contained 0.15 mg/ml of ferrichrysin.

### <Formulation Example 30: Soup>

40 mg of ferrichrysin was added to 100 ml of a thick soup (potage) and then the mixture was stirred well to obtain an iron-fortified soup. The soup contained 0.4 mg/ml of ferrichrysin.

### INDUSTRIAL AVAILABILITY

The iron supplementing agent of the present invention is suitable for use in preventing, improving, or treating the symptoms of iron deficiency such as iron deficiency anemia. The food additive of the present invention is suitable for use in functional foods and foods for specific health uses for the purpose of iron supplementation or preventing or improving iron deficiency anemia.

## Claims

1. An iron supplementing agent comprising a siderophore and iron (III) ions.

2. An iron supplementing agent according to Claim 1, wherein a siderophore and iron (III) ions are contained in the form of a chelate complex.

3. An iron supplementing agent according to Claim 1 or 2, wherein the siderophore incorporates hydroxamic acids.

4. An iron supplementing agent according to Claim 3, wherein the chelate complex is a ferrichrome.

5. An iron supplementing agent according to Claim 4, wherein the ferrichrome is ferrichrysin.

6. An agent for preventing or treating iron deficiency anemia, comprising a siderophore and iron (III) ions.

7. An agent for preventing or treating iron deficiency anemia according to Claim 6, wherein a siderophore and iron (III) ions are contained in the form of a chelate complex.

8. An agent for preventing or treating iron deficiency anemia according to Claim 6 or 7, wherein the siderophore incorporates hydroxamic acids.

9. An agent for preventing or treating iron deficiency anemia according to Claim 8, wherein the chelate complex is a ferrichrome.

10. An agent for preventing or treating iron deficiency anemia according to Claim 9, wherein the ferrichrome is ferrichrysin.

11. A food additive for iron supplementation comprising a siderophore and iron (III) ions.

12. A food additive for preventing or improving iron deficiency anemia comprising a siderophore and iron (III) ions.

13. A food composition comprising a siderophore and iron (III) ions.

14. A food composition according to Claim 13, wherein a siderophore and iron (III) ions are contained in the form of a chelate complex.

15. A food composition according to Claim 13 or 14, wherein the food composition is in solid form, and contains 0.1 to 5 mg/g of the siderophore and iron (III) ions in terms of a chelate complex.

16. A food composition according to Claim 13 or 14, wherein the food composition is in liquid form, and contains 0.05 to 10 mg/ml of the siderophore and iron (III) ions in terms of a chelate complex.

17. A food composition according to Claim 16, wherein the food is a beverage selected from the group consisting of liquors, teas, coffees, sports drinks, refreshment drinks, dairy drinks, and soups.

18. A food composition according to any one of Claims 13 to 17 for use in iron supplementation.

19. A food composition according to any one of Claims 13 to 17 for use in preventing or improving iron deficiency anemia.

20. A food composition according to any one of Claims (i) to (vi) below, each comprising a siderophore and iron (III) ions:
(i) a food composition which has the function of supplementing iron, and carries an indication specifying a use for supplementing iron;
(ii) a food composition which has the function of assisting iron, and carries an indication specifying a use for assisting iron;
(iii) a food composition which has the function of fortifying iron, and carries an indication specifying a use for fortifying iron;
(iv) a food composition which has the function of adding iron, and carries an indication specifying a use for adding iron;
(v) a food composition which has the function of maintaining bodily iron content at a normal level, and carries an indication specifying a use for maintaining bodily iron content at a normal level; and
(vi) a food composition which has the function of eliminating or alleviating iron deficiency in the body, and carries an indication specifying a use for eliminating or alleviating iron deficiency in the body.

21. An iron supplementing method comprising administering a composition containing a siderophore and iron (III) ions to a human.

22. An iron supplementing method according to Claim 21, wherein 20 to 170 mg of a siderophore and iron (III) ions in terms of a chelate complex is administered per day.

23. A method of preventing or treating iron deficiency anemia comprising administering a composition containing a siderophore and iron (III) ions to a human.

24. A method of preventing or treating iron deficiency anemia according to Claim 23, wherein 20 to 170 mg of a siderophore and iron (III) ions in terms of a chelate complex is administered per day.

25. A method of adding a composition containing a siderophore and iron (III) ions to a food.

26. Use of a composition containing a siderophore and iron (III) ions as an iron supplementing agent.

27. Use of a composition containing a siderophore and iron (III) ions as an agent for preventing or treating iron deficiency anemia.

28. Use of a composition containing a siderophore and iron (III) ions as a food additive for iron supplementation.

29. Use of a composition containing a siderophore and iron (III) ions as a food additive for preventing or improving iron deficiency anemia.

30. Use of a composition containing a siderophore and iron (III) ions as a food composition for iron supplementation.

31. Use of a composition containing a siderophore and iron (III) ions as a food composition for preventing or improving iron deficiency anemia.
